(19) Europäisches Patentamt
European Patent Office
Office européen des brevets

(11) Veröffentlichungsnummer: **0 003 304**
**B1**

(12) **EUROPÄISCHE PATENTSCHRIFT**

(45) Veröffentlichungstag der Patentschrift: **25.11.81**

(21) Anmeldenummer: **79100106.8**

(22) Anmeldetag: **15.01.79**

(51) Int. Cl.³: **C 07 C 99/00,**
C 07 C 101/16 //A01N37/44

(54) **Verfahren zur Herstellung von N-substituierten 2,6-Dialkylanilinen.**

(30) Priorität: **19.01.78 DE 2802211**

(43) Veröffentlichungstag der Anmeldung:
**08.08.79 Patentblatt 79/16**

(45) Bekanntmachung des Hinweises auf die Patenterteilung:
**25.11.81 Patentblatt 81/47**

(84) Benannte Vertragsstaaten:
**BE CH DE FR GB NL**

(56) Entgegenhaltungen:
DE - A - 2 350 944
DE - A - 2 405 510
DE - A - 2 515 113
DE - A - 2 632 437
DE - A - 2 648 074
DE - A - 2 722 383
US - A - 3 942 972
US - A - 3 946 044

(73) Patentinhaber: **BASF Aktiengesellschaft**
**Carl-Bosch-Strasse 38**
**D-6700 Ludwigshafen (DE)**

(72) Erfinder: **Plath, Peter, Dr.**
**Berner Weg 24**
**D-6700 Ludwigshafen (DE)**
Erfinder: **Rohr, Wolfgang, Dr.**
**Gontardstrasse 4**
**D-6800 Mannheim 1 (DE)**
Erfinder: **Goetz, Norbert, Dr.**
**Schoefferstrasse 25**
**D-6520 Worms (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 304 B1

# Verfahren zur Herstellung von N-substituierten 2,6-Dialkylanilinen

Die Erfindung betrifft ein Verfahren zur Herstellung von N-substituierten 2,6-Dialkylanilinen durch Umsetzung von 2,6-Dialkylanilinen mit Carbonylverbindungen und anschließende katalytische Hydrierung mit Wasserstoff.

Es ist aus J. Chem. Soc., Band 1961, Seiten 3 303 bis 3 308 bekannt, daß man 2,6-Dimethylanilin und Äthyl-$\alpha$-brompropionat in Benzol zu Äthyl-$\alpha$-(2,6-dimethylanilino)-propionat umsetzen kann. Obwohl die Umsetzungszeit 96 Stunden beträgt, kann nur eine Ausbeute von 37 Prozent erzielt werden. Wie die Chemischen Berichte, Band 22 (1889), Seiten 1 792 bis 1 795, zeigen, ist aber die Ausbeute nahezu quantitativ, wenn die beiden sterisch hindernden Substituenten in 2,6-Stellung fehlen, bzw. mit Anilin als Ausgangsstoff umgesetzt wird. Eine Umsetzung von Anilin im Vergleich zu Ortho-Methylanilin während 12 Stunden bei 120 bis 125°C mit Äthyl-2-chlorpropionat unter Zusatz von Natriumacetat (C. R. Acad. Sc. Paris, Band 264 (Serie C, 1967), Seiten 1 864 bis 1 865) zeigt Ausbeuten von 40 Prozent bezüglich Anilin und 20 Prozent bezüglich Ortho-Methylanilin und damit den Einfluß schon eines Ortho-Substituenten. Die gleiche Umsetzung mit der Methylgruppe in Meta- oder Para- anstelle in Ortho-Stellung ergibt Ausbeuten von 33 und 57 Prozent. Die Ausbeute wird somit größer, je weiter der Substituent von der Aminogruppe entfernt ist.

Führt man die Umsetzung von 6 g-Mol 2,6-Dimethylanilin mit 18 g-Mol $\alpha$-Brompropionsäuremethylester unter Zusatz von 6,6 g-Mol Natriumbicarbonat durch, so verbessert sich die Ausbeute nach Angaben in der deutschen Offenlegungsschrift 2 350 944 (Seite 15, Beispiel 1) auf 79,6 Prozent; die Umsetzungszeit beträgt insgesamt mehr als 19 Stunden.

Alle diese Verfahren sind mit Bezug auf einfachen und wirtschaftlichen Betrieb, Ausbeute und Aufarbeitung des Endstoffs unbefriedigend.

Das US-Patent 3 946 044 zeigt Herstellung und Verwendung von 1,3-Dioxolanen und ihren Derivaten, z.B. N-$\alpha$-Chloroacetyl-N-(1,3-dioxolan-2-ylmethyl)-2-ethyl-6-methylanilin. Schiffsche Basen treten nicht als Zwischenprodukte auf. In der DE—OS 2 405 510 werden a) mehrkernige Verbindungen (II) mit $\alpha$-Haloalkanoylchloriden oder b) Aniline (IV) mit halogenierten Heterocyclen (V) oder c) 2 Ringe enthaltende Verbindungen VIII hydriert oder d) VIII wird aus substituiertem Anilin und Furfuralverbindungen hergestellt. Zwar wird hier eine Schiffsche Base erhalten, aber nur mit einem aromatischen bzw. heterocyclischen Ringkörper.

Es wurde nun gefunden, daß man N-substituierte 2,6-Dialkylaniline der Formel

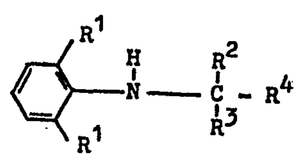

I,

worin die einzelnen Reste $R^1$ und $R^3$ gleich oder verschieden sein können und jeweils einen Alkylrest mit 1 bis 7 Kohlenstoffatomen bedeuten, $R^2$ ein Wasserstoffatom bezeichnet, $R^4$ für den Rest

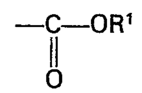

steht, worin $R^1$ die vorgenannte Bedeutung hat, durch Umsetzung von aromatischen Aminen mit Carbonylverbindungen und Hydrierung der gebildeten Schiffschen Base, vorteilhaft erhält, wenn man 2,6-Dialkylaniline der Formel

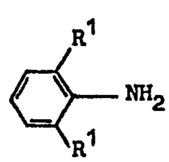

II,

worin $R^1$ die vorgenannte Bedeutung hat, bei einer temperatur von 40 bis 200°C mit Carbonylverbindungen der Formel

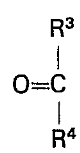

III,

worin $R^3$ und $R^4$ die vorgenannte Bedeutung besitzen, umsetzt und in einem zweiten Schritt die so gebildete Schiffsche Base mit Wasserstoff in Gegenwart eines Hydrierkatalysators bei einer Temperatur von 0 bis 250°C hydriert.

Die Umsetzung kann für den Fall der Verwendung von 2,6-Dimethylanilin und Brenztraubensäuremethylester durch die folgenden Formen wiedergegeben werden:

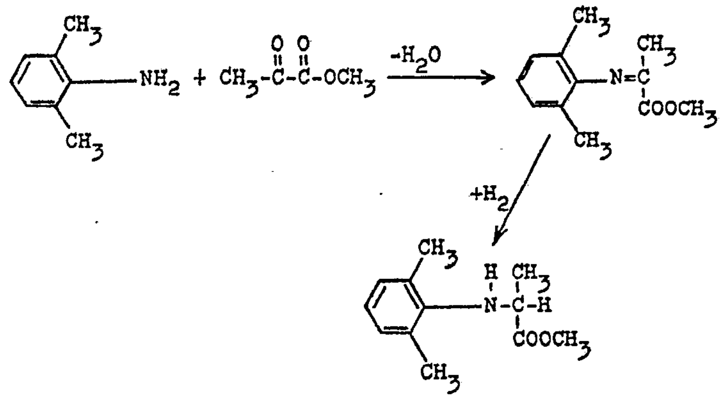


Im Hinblick auf die bekannten Verfahren liefert das Verfahren nach der Erfindung auf einfacherem und wirtschaftlicherem Wege N-substituierte 2,6-Dialkylaniline in besserer Ausbeute und Reinheit. Da weder Halogenwasserstoffsäure gebildet noch basische Verbindungen zu ihrer Bindung zugesetzt werden, ist der Betrieb einfacher und werden Abwasserprobleme infolge andernfalls anfallender hoher Salzmengen, gerade im industriellen Maßstab, vermieden. Das Verfahren ist umweltfreundlicher. Da beide Stufen der Reaktion rasch verlaufen, ist die Umsetzungszeit beider Stufen insgesamt vergleichsweise kürzer und somit die Raum-Zeit-Ausbeute im Gesamtbetrieb besser. Das Verfahren kann vorteilhaft einbadig durchgeführt werden.

Alle diese vorteilhaften Ergebnisse sind im Hinblick auf den Stand der Technik überraschend. Man hätte in Anbetracht der beiden sterisch hindernden Substituenten in 2- und 6-Stellung eine Behinderung oder die Undurchführbarkeit der Kondensation und Hydrierung erwarten müssen.

So zeigt J. Am. Chem. Soc., Band 65 (1943), Seiten 1 017 und 1 018, daß schon 2-Naphthylamin, das in den beiden Ortho-Stellungen unsubstituiert ist, bei der Umsetzung mit Oxalessigsäureäthylester nur 41 Prozent Ausbeute an der Schiffschen Base Diäthyl-2-naphthyliminobernsteinsäureester liefert; Die Umsetzungszeit beträgt 48 Stunden. Der Endstoff wurde zweimal aus Äthanol umkristallisiert, bevor er in einer gesonderten Reaktion zum Diäthyl-2-naphthylaminobernsteinsäureester hydriert wurde. Da bei dem erfindungsgemäßen Verfahren im allgemeinen keine Reinigung der Schiffschen Base stattfindet, sondern einbadig, gegebenenfalls unter Austausch der Lösungsmittel von der ersten zur zweiten Stufe, umgesetzt wird, ist die hohe Ausbeute an reinem Endstoff auch mit Bezug auf diese Veröffentlichung überraschend. Im Hinblick auf das Verhalten von Anilinen bei der Umsetzung mit Acrylnitril waren ebenfalls zumindest nur geringe Ausbeuten an dem erfindungsgemäßen Endstoff zu erwarten. Denn unsubstituiertes Anilin (Houben-Weyl, Methoden der Organischen Chemie, Band 11/1, Seite 275) ergibt eine Ausbeute von 53,4 Prozent an β-Anilinopropionitril, während Amine, in denen beide Ortho-Stellungen besetzt sind, überhaupt nicht reagieren.

Auch im Hinblick auf US-Patent 3 946 044 und DE—OS 2 405 510 besitzen die erfindungsgemäßen Ausgangsstoffe völlig andere Struktur, insbesondere keine mehrkernige Struktur. Die vorteilhaften Ergebnisse sind daher auch im Hinblick auf diese beiden Druckschriften überraschend.

Ausgangsstoff III kann mit Ausgangsstoff II in stöchiometrischer Menge oder im Überschuß jeder Komponenten zur anderen, vorteilhaft in einer Menge von 0,8 bis 2, vorzugsweise 1 bis 1,2 Mol Ausgangsstoff III je Mol Ausgangsstoff II umgesetzt werden. Bevorzugte Ausgangsstoffe II und III und dementsprechend bevorzugte Endstoffe I sind solche, in deren Formeln die die einzelnen $R^1$ und $R^3$ gleich oder verschieden sein können und jeweils einen Alkylrest mit 1 bis 4 Kohlenstoffatomen, bedeuten, $R^2$ auch ein Wasserstoffatom bezeichnen kann, $R^4$ für den Rest

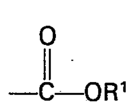

steht, worin $R^1$ die letztgenannte der vorgenannten Bedeutungen hat. Treten im Endstoff I 3 Reste $R^1$ auf, so können die einzelnen Reste $R^1$ gleich oder verschieden sein. Die vorgenannten Reste können

noch durch unter den Reaktionsbedingungen inerte Gruppen, z.B. Alkylgruppen mit 1 bis 4 Kohlenstoffatomen, substituiert sein.

Beispielsweise kommen folgende Ausgangsstoffe II in Betracht: 2,6-Dimethyl-, 2,6-Diäthyl-, 2,6-Di-n-propyl, 2,6-Diisopropyl-, 2,6-Di-n-butyl-, 2,6-diisobutyl-, 2,6-Di-sek.-butyl-, 2,6-Di-tert.-butylanilin, 2-Methyl-6-äthyl-, 2-Methyl-6-propyl-, 2-Methyl-6-isopropyl-, 2-Methyl-6-butyl-, 2-Methyl-6-isobutyl-, 2-Methyl-6-sek.-butyl-, 2-Methyl-6-tert.-butyl-anilin, 2-Äthyl-6-propyl-, 2-Äthyl-6-isopropyl-, 2-Äthyl-6-butyl-, 2-Äthyl-6-isobutyl-, 2-Äthyl-6-tert.-butyl-anilin.

Folgende Ausgangsstoffe III kommen in Frage: 2-Oxopropionsäure-, 2-Oxobuttersäure-, 2-Oxovaleriansäure-, 2-Oxocapronsäure-, 2-Oxocaprylsäure-methylester und entsprechende Äthyl-, Propyl-, Isopropyl-, Butyl-, Isobutyl-, sek.-Butyl-, tert.-Butyl-ester;

Die Umsetzung wird im ersten Schritt bei einer Temperatur von 40 bis 200, vorzugsweise von 50 bis 100°C, drucklos oder unter Druck, kontinuierlich oder diskontinuierlich durchgeführt. Zweckmäßig verwendet man unter den Reaktionsbedingungen inerte Lösungsmittel. Als Lösungsmittel kommen z.B. in Frage: aromatische Kohlenwasserstoffe, Z.B. Toluol, Äthylbenzol, o- m-, p-Xylol, Isopropylbenzol, Methylnaphthalin; aliphatische oder cycloaliphatische Kohlenwasserstoff, z.B. Heptan, Pinan, Nonan, Benzinfraktionen innerhalb eines Sidepunktintervalls von 70 bis 190°C, Cyclohexan, Methylcyclohexan, Dekalin, Hexan, Ligroin, 2,2,4-Trimethylpentan, 2,2,3-Trimethylpentan, 2,2,3-Trimethylpentan, Octan; und entsprechende Gemische. Zweckmäßig verwendet man das Lösungsmittel in einer Menge von 150 bis 10 000 Gewichtsprozent, vorzugsweise von 300 bis 600 Gewichtsprozent, bezogen auf Ausgangsstoff II.

Die Reaktion kann wie folgt durchgeführt werden: Ein Gemisch von Ausgangsstoff II, III und Lösungsmittel wird während 2 bis 5,5 Stunden bei der Reaktionstemperatur gehalten. Vorteilhaft wird man das gebildete Wasser noch während der Reaktion kontinuierlich, z.B. durch azeotrope Destillation mit einem geeigneten Lösungsmittel wie Cyclohexan, entfernen. Dann kann die Schiffsche Base zwar aus dem Gemisch in üblicher Weise, z.B. durch fraktionierte Destillation, abgetrennt und mit der isolierten, gereinigten Base der zweite Schritt der Umsetzung durchgeführt werden. Diese Arbeitsweise wird man aber in der Regel schon aus wirtschaftlichen Gründen nicht wählen, sondern vorteilhaft nach der Reaktion des ersten Schrittes das Lösungsmittel abdestillieren, ein für den zweiten Schritt geeignetes Lösungsmittel zusetzen und den zweiten Reaktionsschritt bei der Reaktionstemperatur durchführen.

Die Umsetzung des zweiten Schrittes wird bei einer Temperatur von 0 bis 250°C, vorzugsweise von 40 bis 190°C, drucklos oder unter Druck, kontinuierlich oder diskontinuierlich durchgeführt. Zweckmäßig verwendet man unter den Reaktionsbedingungen inerte Lösungsmittel wie Alkanole und Cycloalkanole wie n-Butanol, Isobutanol, tert.-Butanol, Glykol, Glycerin, n-Propanol, Isopropanol, Amylalkohol, Cyclohexanol, 2-Methyl-4-pentanol, Äthylenglykolmonoäthyläther, 2-Äthyl-hexanol, Methylglykol und insbesondere Äthanol, Methanol; cyclische Äther wie Tetrahydrofuran oder Dioxan; die schon für den ersten Reaktionsschritt genannten Lösungsmittel. Bevorzugt kommen die schon für den ersten Schritt genannten Lösungsmittelmengen in Betracht.

Die Umsetzung wird in Gegenwart von Wasserstoff, zweckmäßig im Überschuß, im allgemeinen in einer Menge von 3 bis 25, vorzugsweise von 3 bis 50 Molprozent, bezogen auf Ausgangsstoff II, durchgeführt. Mann kann den Wasserstoff kontinuierlich oder diskontinuierlich der Reaktion zuführen und/oder den Katalysator selbst nach einer bestimmten Reaktionszeit wieder frisch mit Wasserstoff beladen. Bevorzugte Hydrierkatalysatoren sind im allgemeinen ein oder mehrere Metalle mit einer Ordnungszahl zwischen 24 und 29, in der Regel Kobalt-, Kupfer-, Mangan- und/oder Nickelkatalysatoren z.B. entsprechende Sinterkatalysatoren. Die Metalle können im Katalysator auch in Form ihrer Oxide und/oder im Gemisch mit Phosphorsäure vorliegen.

Vorteilhafte Katalysatoren der genannten Art enthalten zwischen 3 und 30 Gewichtsprozent Kupfer, 0,5 und 10 Gewichtsprozent Mangan und 10 bis 80 Gewichtsprozent Kobalt oder Nickel. Hierzu treten gegebenenfalls 0,1 bis 5 Gewichtsprozent Phosphorsäure, bezogen auf die Metallmenge. Der Hydrierkatalysator wird in der Regel bei der Umsetzung in einer Menge von 5 bis 30 Gewichtsprozent, bezogen auf Ausgangsstoff II, verwendet. Er kann im Gemisch mit einem für die Umsetzung geeigneten Trägermaterial, z.B. Siliciumdioxid, zur Anwendung gelangen, wobei zweckmäßig die Menge des Katalysators 10 bis 40 Gewichtsprozent des Gemisches von Katalysator und Träger beträgt. In der Regel werden dem Reaktionsgemisch am Anfang und im Verlauf der umsetzung solche Mengen an Wasserstoff zugeführt, daß sich bei der Umsetzungstemperatur stets ein entsprechender Reaktionsdruck, zweckmäßig zwischen 150 und 300 bar, einstellt.

Die Umsetzung wird im allgemeinen bei einer Temperatur von 22 bis 200°C, vorzugsweise von 25 bis 160°C, diskontinuierlich oder kontinuierlich durchgeführt. Zur entsprechenden Druckeinstellung können auch inerte Gase wie Stickstoff, verwendet werden.

Zweckmäßig verwendet man ebenfalls Kupferchromitkatalysatoren, z.B. entsprechende Kupfer-Chromoxitkatalysatoren wie die von H. Adkins verwendeten Kupferchromite. Sie enthalten beispielsweise Kupfer-Chrom-Spinell ($CuCr_2O_4$) oder Gemische im Verhältnis 5 CuO : 4 $Cr_2O_3$ bzw. gehen von solchen Verbindungen aus und können noch andere Oxide, hauptsächlich die der Erdalkalimetalle wie Barium, Calcium oder Magnesium enthalten. Das Kupferchromit kann zweckmäßig durch Ausfällung von Kupfer-Ammoniumchromat aus Kupfernitrat-Ammoniumchromatlösungen, Zersetzung des

Kupfer-Ammoniumchromats bei erhöhter Temperatur und Behandeln des so erhalten Kupfer-Herstellung von Kupferchromitkatalysatoren wird auf loc. cit., Band 4/2, Seiten 180 bis 183 Ebenenfalls kann man das Kupfer-Ammoniumchromat bei niederer Temperatur zersetzen und die Reduktion des Kupferchromits in Methanol mit Wasserstoff bei 100 bis 200 bar vornehmen. Bezüglich der Herstellung von Kupferchromitkatalysatoren wird auf Houben-Weyl, loc. cit., Band 4/2, Seiten 180 bis 183 und Journal of Applied Chemistry, Band 5 (1955), Seiten 289 bis 295, verwiesen. Im allgemeinen wird das erfindungsgemäße Verfahren in Gegenwart von Kupferchromitkatalysatoren bei einer Temperatur von 22 bis 200°C, vorzugsweise 90 bis 190°C, diskontinuierlich oder kontinuierlich unter Druck, zweckmäßig 50 bis 300, insbesondere von 140 bis 280 bar, durchgeführt.

Vorteilhaft sind auch Kobalt- oder Nickel-Sinterkatalysatoren, die bis zu 30 Gewichtsprozent Kupfer, Mangan, Eisen und/oder Chrom enthalten können; vorzugsweise gelangen Raney-Nickel und Raney-Koblat zur Verwendung. Solche Hydirerkatalysatoren werden in der Regel in Mengen von 0,5 bis 50 Gewichtsprozent, bezogen auf Ausgangsstoff II, zugesetzt. Die Umsetzung findet bie einem Reaktionsdruck zwischen 50 und 300 bar, kontinuierlich oder diskontinuierlich, statt; vorgenannte Reaktionstemperaturen kommen in Frage.

Man kann die Umsetzung auch in Gegenwart von Palladium und/oder seinen Verbindungen, im allgemeinen in einer Menge von 0,01 bis 5 Gewichtsprozent, vorzugsweise 0,1 bis 2 Gewichtsprozent Palladium als feinverteiltes Metall und/oder berechnet als Palladium in Gestalt seiner feinverteilten Verbindungen, bezogen auf Ausgangsstoff II, durchführen. So können z.B. als Isomerisierungskatalysatoren Palladiummohr, Palladiumpulver, Palladiumbromid, -arsenid, -cyanid, -chlorid, -nitrat, -jodid, -oxid, sulfid, -sulfat oder Komplexsalze wie Tetrachlorpalladate, Tetraamin- oder Diaminpalladiumchloride, Hexachlorpalladate, verwendet werden. Vorteilhaft können die genannten Katalysatoren auch in bekannter Weise auf Träger, z.B. Aktivkohle, Bariumsulfat, Kieselgel oder Zeolithe aufgebracht und solche Trägerkatalysatoren für die Isomerisierung verwendet werden. Die Herstellung solcher Trägerkatalysatoren kann sich in beliebiger Weise, z.B. durch Tränken des Trägers, mit entsprechenden Lösungen der Palladiumsalze, durch Verkneten bzw. Mischen unter Vermahlen der Komponenten, vollziehen. Bezüglich Details der Herstellung von Katalysatoren, insbesondere Trägerkatalysatoren, wird auf Houben-Weyl, Methoden der Organischen Chemie, Band 4/2, Seiten 137 ff, verwiesen. In entsprechender Weise können auch Platin und/oder seine Verbindungen, z.B. Platinoxid, als Hydrierkatalysatoren verwendet werden. Die Umsetzung wird mit Wasserstoff, zweckmäßig in einer Menge von 1 bis 50, vorzugsweise von 5 bis 25 Molprozent, bezogen auf Ausgangsstoff II, durchgeführt. Man kann den Wasserstoff kontinuierlich oder diskontinuierlich der Reaktion zuführen und/oder den Katalysator selbst nach einer bestimmten Reaktionszeit wieder frisch mit Wasserstoff beladen. Man setzt den Ausgangsstoff II in der Regel bei einer Temperatur zwischen 20 und 200°C, vorzugsweise zwischen 50 und 150°C, drucklos oder unter Druck, z.B. 10 bis 100 bar, um.

Die Umsetzung des zweiten Schrittes kann wie folgt durchgeführt werden: Man gibt das Lösungsmittel in den Reaktor, der das vom Lösungsmittel des ersten Schrittes befreite Reaktionsgemisch enthält, setzt den Hydrierkatalysator zu und spült den Reaktionsraum mit Stickstoff. Dann wird Wasserstoff bis zu vorgenanntem Reaktionsdruck eingepreßt. Nun wird das Reaktionsgemisch auf vorgenannte Temperatur gebracht und solange bei dieser Temperatur unter Einleitung von weiterem Wasserstoff gehalten, bis kein Wasserstoff mehr durch die Reaktion verbraucht wird; im allgemeinen beträgt diese Reaktionszeit von 2 bis 8 Stunden, zweckmäßig bei tieferer Temperatur zwischen 4 und 12 Stunden, bei höherer Temperatur 2 bis 6 Stunden. Nun wird das Reaktionsgemisch abgekühlt und filtriert. Aus dem Filtrat wird der Endstoff nach den üblichen Methoden, z.B. durch Eindampfen des Filtrats, abgetrennt.

In einer bevorzugten Ausführungsform wird die Umsetzung einbadig durchgeführt, indem man in vorgenannter Weise den ersten Schritt der Reaktion bei der Reaktionstemperatur des ersten Schrittes durchführt, Hydrierkatalysator zugibt und den zweiten Schritt der Reaktion, gegebenenfalls unter Temperaturhöhung, durchführt.

Die nach dem Verfahren der Erfindung herstellbaren Verbindungen sind wertvolle Ausgangsstoffe für die Herstellung von Farbstoffen und Schädlingsbekämpfungsmitteln. Insbesondere können sie durch Acylierung des Stickstoffatoms in N-substituierte Verbindungen umgewandelt werden, die Wirkstoffe zur Bekämpfung von phytopathogenen Pilzen und Bakterien darstellen. Diese N-Acylverbindungen besitzen sowohl präventive als auch kurative Wirkung gegen phytopathogene Pilze an Kulturpflanzen wie beispielsweise Getreide, Mais, Reis, Gemüse, Zuckerrüben, Soja, Erdnüssen, an Obstbäumen, Zierpflanzen, vor allem aber an Reben, Hopfen, Gurkengewächsen (Gurken, Kürbis, Melonen) und Solanaceen wie Kartoffeln, Tabak und Tomaten.

Mit diesen Wirkstoffen können an Pflanzen oder Pflanzenteilen (Früchte, Blüten, Laubwerk, Stengel, Knollen, Wurzeln) auftretende Pilze eingedämmt oder vernichtet werden, wobei auch später zuwachsende Pflanzenteile von derartigen Pilzen verschont bleiben. Sie können ferner zur Behandlung von Saatgut (Früchte, Knollen, Körner) und Pflanzenstecklingen zum Schutz vor Pilzinfektionen eingesetzt werden. In diesem Zusammenhang wird auf die deutschen Offenlegungsschriften 2 350 944, 2 515 091, 2 515 113, 2 513 730, 2 513 732, 2 513 788 und 2 513 789 und die dort beschriebenen Verwendungen sowie auf die zum Stand der Technik genannten Veröffentlichungen verwiesen.

Die in den folgenden Beispielen aufgeführten Teile bedeuten Gewichtsteile.

Beispiele 1

121 Teile 2,6-Dimethylanilin in 400 Teilen Cyclohexan werden in einem Rührkessel mit 128 Teilen Brenztraubensäureäthylester versetzt. Man erhitzt das Gemisch während 3,5 Stunden bei 70°C, wobei 18 Teile Wasser azeotrop abdestilliert werden. Man destilliert Cyclohexan ab, setzt 400 Teile Tetrahydrofuran und 30 Teile Raney-Nickel zu, leitet das Gemisch in einen nachfolgenden Autoklaven, spült den Autoklaven dreimal mit Stickstoff und gibt Wasserstoff bis zu einem Druck von 200 bar zu. Nach 3 Stunden ist der Wasserstoffdruck auf 175 bar gesunken. Der Wasserstoffdruck wird nun erneut auf 200 bar erhöht; anschließend wird der Autoklav auf 50°C erwärmt. In den folgenden 3 Stunden werden noch 5 bar Wasserstoff aufgenommen, insgesamt werden 2,2 Teile Wasserstoff verbraucht. Nach Entspannen des Autoklaven, Abtrennung des Nickels durch Filtration unter Stickstoff und Destillation des Filtrats erhält man 208 Teile (94% der Theorie) 2-(2',6'-Dimethylphenylamino)-propionsäureäthylester vom Kp (0,3 mbar) 101°C.

Beispiel 2

Die Umsetzung wird analog Beispiel 1, jedoch einbadig ohne Zusatz von Tetrahydrofuran und ohne Abtrennung des Cyclohexans durchgeführt. Man erhält 200 Teile (90,5% der Theorie) 2-(2',6'-Dimethylphenylamino)-propionsäureäthylester vom Kp (0,3 mbar) 101°C.

Beispiele 3 bis 6

Analog Beispiel 1 werden die folgenden Umsetzungen durchgeführt, deren Reaktionsbedingungen die Tabellen 1 und 2 zeigen.

TABELLE 1

1. Reaktionsschritt (Herstellung der Schiffschen Base)

| Beispiel | Ausgangsstoff II | Teile Ausgangsstoff II | Ausgangsstoff III | Teile Ausgangsstoff III | Lösungsmittel (Teile) | Reaktionszeit (Stunden) | Temperatur (°C) |
|---|---|---|---|---|---|---|---|
| 3 | 2,6-Dimethylanilin | 121 | Brenztraubensäuremethylester | 112 | Cyclohexan (400) | 4,5 | 70 |
| 4 | 2,6-Dimethylanilin | 121 | Brenztraubensäuremethylester | 112 | Toluol (400) | 4 | 85 |
| 5 | 2-Methyl-6-äthylanilin | 135 | Brenztraubensäuremethylester | 112 | Cyclohexan (300) | 5 | 70 |
| 6 | 2-Methyl-6-äthylanilin | 135 | Brenztraubensäureäthylester | 128 | Toluol (300) | 5 | 85 |

TABELLE 2 – 2. Reaktionsschritt (Hydrierung)

| Beispiel | Lösungsmittel (Teile) | Katalysator (Teile) | $H_2$-Druck (bar) | Verbrauch in Teilen $H_2$ | Temperatur (°C) | Zeit (Stunden) | Endstoff | Ausbeute in Teilen (% der Theorie) | Kp (°C) in mbar |
|---|---|---|---|---|---|---|---|---|---|
| 3 | Tetrahydrofuran (400) | Raney-Nickel (30) | 200 | 2,1 | 25 | 8 | 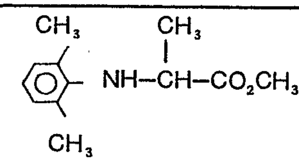 | 188 (91) | 87–89/0,2 |
| 4 | Methanol (200) | Palladium (5 Gew. % auf Ativkohle) (10) | 100 | 2,1 | 70 | 6 | 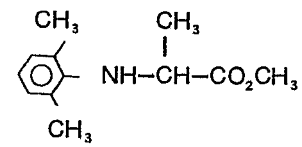 | 190 (92) | 87–89/0,2 |
| 5 | Tetrahydrofuran (400) | Raney-Nickel (30) | 200 | 2,1 | 30 | 8 | 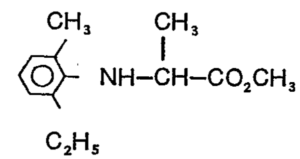 | 197 (89) | 91–93/0,2 |
| 6 | Äthanol (500) | Raney-Nickel (20) | 150 | 2,1 | 90 | 3 | 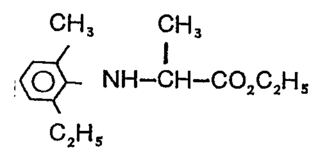 | 211 (90) | 95–97/0,2 |

Beispiel 7

(Biologische Verwendung)

Als Kulturgefäße dienten Plastikblumentöpfe mit 300 $cm^3$ Inhalt und lehmiger Sand mit etwa 1,5 Prozent Humus als Substrat. Die Samen der Testpflanzen entsprechend Tabelle 3 werden nach Arten getrennt flach eingesät. Unmittelbar danach besprüht man die Erdoberfläche mit den Wirkstoffen (Vorauflaufbehandlung). Die Wirkstoffe werden in Wasser als Verteilungsmittel suspendiert (2 kg/ha Wirkstoff in 3 000 Liter Wasser) und mittels fein verteilender Düsen versprüht. Nach dem Aufbringen der Wirkstoffe werden die Gefäße leicht beregnet, um Keimung und Wachstum in Gang zu bringen und auch gleichzeitig die Wirkstoffe zu aktivieren. Danach deckte man die Gefäße mit durchsichtigen Plastikhauben ab, bis die Pflanzen angewachsen sind. Die Abdeckung bewirkt ein gleichmäßiges Keimen der Testpflanzen und verhinderte das Verdampfen leicht flüchtiger Substanzen.

Die Aufstellung erfolgt im Gewächshaus, wobei eine Temperatur von 15 bis 40°C während 6 Wochen eingehalten wird.

TABELLE 3 — Liste der Pflanzennamen

| Lateinische Bezeichnung | Abkürzung in Tabelle | Deutscher Name | Englischer Name |
|---|---|---|---|
| Arachys hypogaea | Arach. hyp. | Erdnuß | peanut (groundnut) |
| Beta vulgaris | Beta vulg. | Zuckerrübe | sugarbeets |
| Bromus spp. | Bromus spp. | Trespenarten | brome spp. |
| Digitaria spp. | Digit. spp. | Fingerhirsen | crabgrass |
| Echinochloa crus galli | Echino. c.g. | Hühnerhirse | barnyardgrass |
| Cyperus spp. (C. ferax Cyperus difformis) | Cyperus spp. | Cypergras (aus Samen) | annual seedges (grown from seeds) |
| Glycine max | Glyc max | Sojabohne | soybeans |
| Triticum aestivum | Tritic. aes | Weizen | wheat |

**Patentanspruch**

Verfahren zur Herstellung von N-substituierten 2,6-Dialkylanilinen der Formel

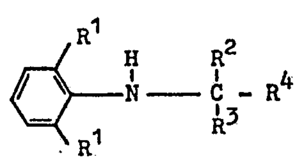 I,

worin die einzelnen Reste $R^1$ und $R^3$ gleich oder verschieden sein können und jeweils einen Alkylrest mit 1 bis 7 Kohlenstoffatomen bedeuten, $R^2$ ein Wasserstoffatom bezeichnet, $R^4$ für den Rest

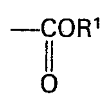

steht, worin $R^1$ die vorgenannte Bedeutung hat, durch Umsetzung von aromatischen Aminen mit Carbonylverbindungen und Hydrierung der gebildeten Schiffschen Base, dadurch gekennzeichnet, daß man 2,6-Dialkylaniline der Formel

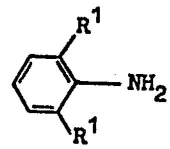

II,

worin $R^1$ die vorgenannte Bedeutung hat, bei einer Temperatur von 40 bis 200°C mit Carbonylverbindungen der Formel

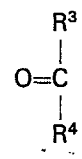

III,

worin $R^3$ und $R^4$ die vorgenannte Bedeutung besitzen, umsetzt und in einem zweiten Schritt die so gebildete Schiffsche Base mit Wasserstoff in Gegenwart eines Hydrierkatalysators bei einer Temperatur von 0 bis 250°C hydriert.

**Claim**

A process for the preparation of an N-substituted 2,6-dialkylaniline of the formula

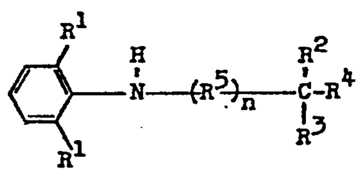

I

where the individual radicals $R^1$ and $R^3$ may be identical or different and each is alkyl of 1 to 7 carbon atoms, $R^2$ is hydrogen, $R^4$ is

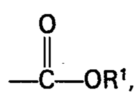

where $R^1$ has the above meaning, by reaction of an aromatic amine with a carbonyl compound and hydrogenation of the resulting Schiff base, characterised in that a 2,6-dialkylaniline of the formula

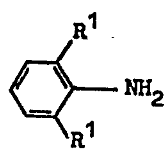

II

where $R^1$ has the above meaning is reacted at a temperature of from 40 to 200°C with a carbonyl compound of the formula

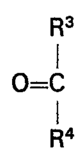

III

where $R^3$ and $R^4$ have the above meanings, and, in a second step, the resulting Schiff base is hydrogenated with hydrogen in the presence of a hydrogenation catalyst at a temperature of from 0 to 250°C.

**Revendication**

Procédé de préparation de dialcoyl-2,6 anilines N-substituées de la formule

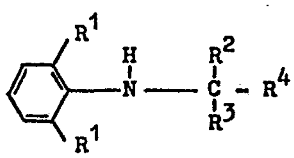

I,

dans laquelle les substituants $R^1$ et le substituant $R^3$ désignent des radicaux alcoyle en $C_1$ à $C_7$, qui peuvent être identiques ou différents, $R^2$ représente un atome d'hydrogène et $R^4$ un groupe

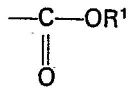

dans lequel $R^1$ possède l'une des significations définies, par réaction d'amines aromatiques avec des composés carbonylés et hydrogénation subséquente de la base de Schiff formée, caractérisé en ce que l'on fait réagir une dialcoyl-2,6 aniline de la formule

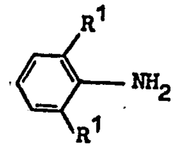

II,

dans laquelle les substituants $R^1$ possèdent les significations définies, à une température entre 40 et 200°C avec un composé carbonylé de la formule

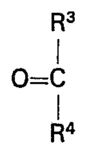

III,

dans laquelle $R^3$ et $R^4$ possèdent les significations définies, puis on soumet, dans un deuxième stade, la base de Schiff formée à une hydrogénation par l'hydrogène en présence d'un catalyseur d'hydrogénation, à une température entre 0 et 250°C.